# EUROPEAN PATENT APPLICATION

(11) **EP 3 950 026 A1**
(43) Date of publication of application: **09.02.2022**
(21) Application number: 20779501.4
(22) Date of filing: 22.03.2020
(51) Int. Cl.: A61M 5/315, A61M 5/20

(54) **PEN INJECTOR**

(30) Priority: 27.03.2019 RU 2019108982
(71) Applicant: Obshchestvo S Organichennoi Otvetstvennostiu «NEXT BIO», Saint-Petersburg, 191144 (RU)
(72) Inventor: RODIONOV, Petr Petrovich, Leningradskaya obl, 187544 (RU); KAZEENKOV, Roman Sergeevich, Samarskaya obl., 445027 (RU); TARASENKO, Fedor Dmitrievich, Saint-Petersburg, 197022 (RU); ZHMAYLO, Mikhail Alexandrovich, Saint- Petersburg, 194358 (RU); KHAFIZOV, Ruslan Ildarovich, g. Surgut, 628416 (RU)
(74) Representative: Pharma Patents International AG
(86) International application number: PCT/RU2020/050053
(87) International publication number: WO 2020/197442

(57) **Abstract**

The present invention relates to an injection syringe pen with an improved dose-setting mechanism. The injection syringe pen comprises a housing provided with a push button; a dose-setting mechanism connected to the push button, wherein dose-setting mechanism is installed in the housing so as to be longitudinally movable in relation thereto; a cylindrically shaped drive installed inside the dose-setting mechanism so as to be longitudinally movable in relation to the housing, wherein the drive is engaged with the dose-setting mechanism; a rod installed in the cylindrically shaped drive; a dose delivery screw installed inside the rod; and an adjusting ring, wherein the adjusting ring is engaged with the dose-setting mechanism and provided with projections on an inner side, the adjusting ring is further rotatably mounted on the rod, and wherein recesses are provided on an outer surface of the rod, and the adjusting ring is mounted on the rod such that the projections of the adjusting ring and the recesses of the rod form a ratchet mechanism.

## Description

### FIELD OF THE INVENTION

The present invention relates to an injection device with an improved dose-setting mechanism.

### BACKGROUND OF THE INVENTION

The invention relates to syringes shaped as a pen or pencil.

Such pen-shaped syringes are used by patients who are required to repeatedly inject drug doses and forced to heal themselves. Patients need to inject a drug dose which may be set individually before each injection or may be set without deleting the previous setting after performing the injection and hold until it is manually adjusted.

Insulin-dependent patients (diabetics) repeatedly face situations when there are no medical workers who could subcutaneously or intramuscularly inject the set dose, some of the patients have a habit to inject insulin or its analogue by themselves. Meanwhile, in many cases diabetics are patients with an impaired vision or defective vision. Thus, such people are required to have a syringe which would allow them to conveniently set a required volume of a liquid drug by using their touch and/or hearing. Moreover, in many cases patients are ready to do so.

The present invention is also intended to support an active lifestyle of the above-mentioned patients. RU 2653780 (priority date: 16 March, 2011; publication date: 14 May, 2018) discloses a syringe pen, comprising: a housing; a push button provided at a proximal end of the housing; a dose-setting mechanism having at least one inner tooth; and a toothed ring on an enlarged head of a drive. However, a main disadvantage of the syringe pen of RU 2653780 is in that parts of the ratchet mechanism are hard to produce due to the teeth provided on the inner side of the dose-setting mechanism. Besides, when the known syringe pen is used, accuracy of dose setting made by the patient is reduced due to a low level of sound and tactile signals.

### SUMMARY OF THE INVENTION

The technical effect achieved by the claimed syringe pen is structural simplification due to simplified production of parts of the ratchet mechanism, and improved accuracy of injection dose setting due to the precise intermeshed connection between the projections of the adjusting ring and the recesses, resulting in an improved sound and tactile effect when setting the dose.

To achieve the technical effect there is provided an injection syringe pen, comprising: a housing provided with a push button; a dose-setting mechanism connected to the push button, wherein the dose-setting mechanism is installed in the housing so as to be longitudinally movable in relation thereto; a cylindrically shaped drive installed inside the dose-setting mechanism so as to be longitudinally movable in relation to the housing, wherein the cylindrically shaped drive is engaged with the dose-setting mechanism; a rod installed in the cylindrically shaped drive; a dose delivery screw installed inside the rod; and an adjusting ring, wherein the adjusting ring is engaged with the dose-setting mechanism and provided with projections on an inner side, the adjusting ring is further rotatably mounted on the rod, and wherein recesses are provided on an outer surface of the rod, and the adjusting ring is mounted on the rod such that the projections of the adjusting ring and the recesses of the rod form a ratchet mechanism.

Simplification of the production of the separate part (the adjusting ring) provided with the projections allows to simplify production of the ratchet mechanism and the syringe pen itself. Further, production of the recesses on the rod also simplifies the technological process as compared to production of projecting section (head) of a drive provided with a toothed ring.

The adjusting ring may be produced by using a pressing method, moulding method or 3D printing. For example, the recesses may be produced by using a bar blade; in particular, the recesses may be provided directly on the rod or on a ring (washer) provided on the rod, thereby decreasing the labor intensity. Likewise, parts having recesses may be produced by using a 3D printer.

The projections of the adjusting ring and the recesses on the rod produce a tight contact therebetween, so that rotation of the ring in relation to the rod results in an increased sound level (vibrations) and provides an increased accuracy of the dose set by the dose setting mechanism.

A length and shape of the projections provided on the ring may be adjusted to control the level of sound and tactile effects when setting the dose.

The adjusting ring may have at least two projections. A number of projections may depend on graduations on the dose-setting mechanism.

The projections may be formed as teeth.

The projections of the adjusting ring may be straight or curved. A curved shape of the projections provides a sound effect only in a single direction of rotation of the dose setting mechanism, e.g. only when the dose is increased.

Straight projections provided on the adjusting ring provide a ratchet intermeshed connection both when increasing and decreasing the injection dose.

The projections of the adjusting ring may have blunt or sharp ends. The sharp projections provide the tight contact with the sharp recesses, thereby allowing an improved sound effect when rotating the adjusting ring in relation to the rod. In case when the projections are provided with blunt ends, the rotation of the dose-setting mechanism results in generation of a muffed sound (i.e. vibration is less sensitive) as compared to the syringe pen structure comprising the adjusting ring with sharp projections.

### BRIEF DESCRIPTION OF THE DRAWINGS

The present invention will be better understood from the following description taken in conjunction with the accompanying drawings, wherein:
FIG. 1 shows the dose-setting mechanism and the cylindrically shaped drive in a disassembled state;
FIG. 2 shows a detailed view of the syringe pen according to the invention;
FIG. 3 shows the housing and the rod in a disassembled state;
FIG. 4 shows a cartridge and the syringe pen according to the invention;
FIG. 5 shows the syringe pen according to the invention, a cartridge and a cap;
FIG. 6 shows the ratchet mechanism in an assembled state;
FIG. 7 shows another view of the ratchet mechanism.

The provided drawings illustrate a ratchet mechanism (an intermeshed connection assembly) in an embodiment of the syringe pen housing. The ratchet mechanism may be also used in other implementations of the syringe pen housing.

The following reference numerals are defined throughout the drawings:
1 - housing;
2 - dose-setting mechanism;
3 - cylindrically shaped drive;
4 - rod (tubular member);
5 - dose delivery screw;
6 - adjusting ring;
7 - projections;
8 - recesses;
9 - cartridge;
10 - cap.

### DETAILED DESCRIPTION OF THE PREFERRED EMBODIMENTS

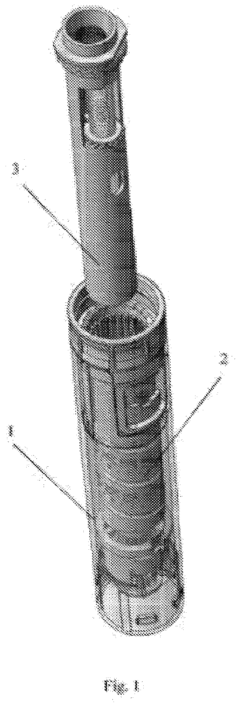

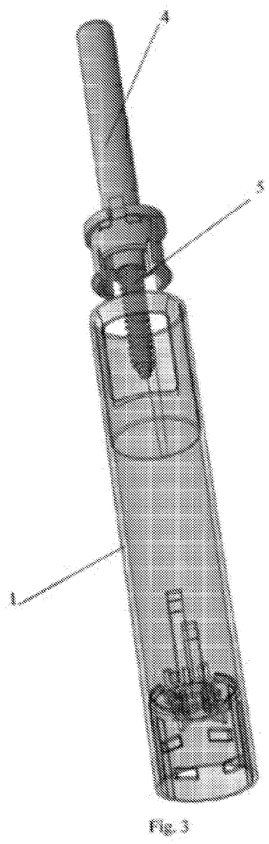

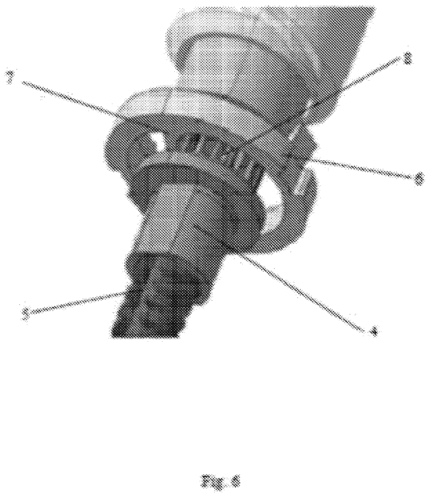

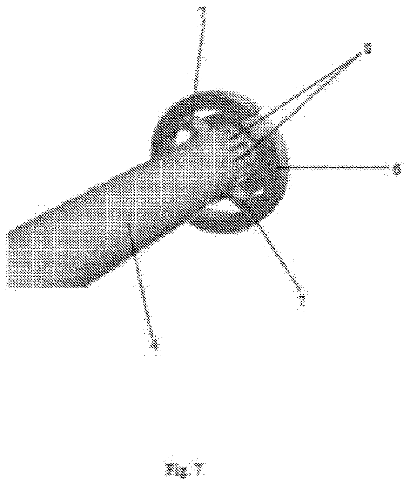

An injection syringe pen (see FIGS. 1-5) comprises a housing 1 provided with a push button (not shown in the drawings). The housing 1 comprises a dose-setting mechanism 2 installed therein, wherein the dose-setting mechanism 2 is connected to the push button and installed in the housing 1 so as to be longitudinally movable in relation to the housing 1. The housing 1 also comprises a cylindrically shaped drive 3 installed in the dose-setting mechanism 2 so as to be longitudinally movable in relation to the housing 1, wherein the drive 3 is engaged with the dose-setting mechanism 2. The housing also comprises a rod 4 (see FIGS. 6, 7) installed in the cylindrically shaped drive 3, a dose delivery screw 5 installed inside the rod 4, and an adjusting ring 6 engaged with the dose-setting mechanism 2 and provided with projections 7 on an inner side, wherein the adjusting ring 6 is installed on the rod 4 so as to be rotatable in relation to the rod 4. Recesses 8 are provided on an outer surface of the rod 4, and the adjusting ring 6 is installed on the rod 4 such that the projections 7 of the adjusting ring 6 and the recesses 8 of the rod 4 form a ratchet mechanism. The adjusting ring 6 may have at least two projections 7. The projections 7 may be formed as teeth. The projections 7 of the adjusting ring 6 may be straight or curved.

The projections 7 of the adjusting ring 6 may be provided with blunt or sharp ends.

The recesses 8 may be circumferentially provided on a surface of the rod 4 directly or on a separate element (e.g. on a washer or ring) which may be positioned on the rod 4.

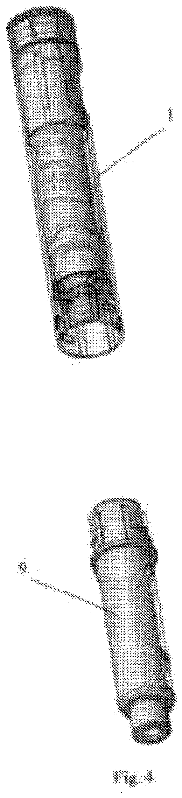

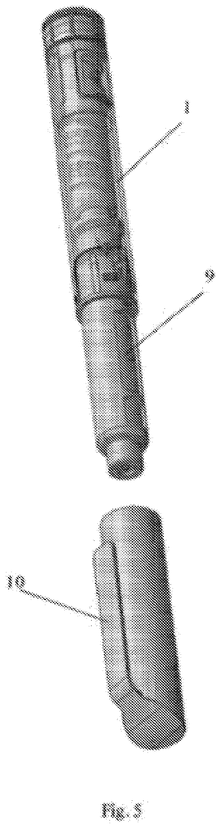

As shown in FIG. 4, the housing 1 of the syringe pen is designed to be connected to a cartridge housing provided with a drug. The syringe pen also comprises a cap 10 covering the cartridge and a needle (not shown in the drawings) when the syringe pen is not used (see FIG. 5).

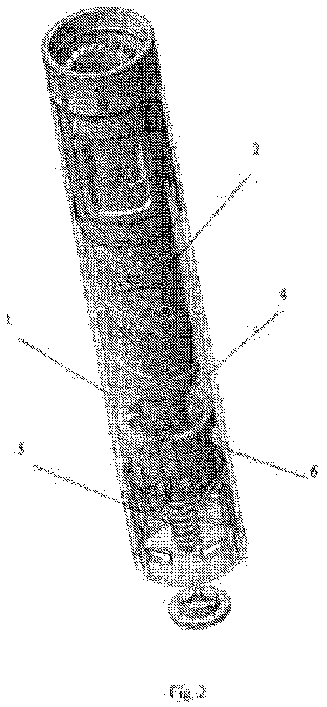

The dose-setting mechanism 2 comprises a rotatable wheel (not shown in the drawings) which may be rotated by the patient to set a required dose. As shown in FIGS. 1-2, the dose-setting mechanism 2 has a plurality of numbers corresponding to dose units which may be seen through a window provided in the housing of the syringe pen (see FIG. 2).

As shown in FIG. 1, the drive is cylindrically shaped and positioned coaxially in the dose-setting mechanism 2. The drive 3 coaxially encloses the rod 4. The rod 4 is provided with a thread on an inner side. The rod 4 coaxially encloses the dose delivery screw 5 which is provided with a thread. The thread of the dose delivery screw 5 mates with the inner thread on the rod 4. The threaded connection between the rod 4 and the dose delivery screw 5 allows the rod 4 to be moved into the cartridge 9 when performing the injection.

The syringe pen having the present structure may be used multiple times if a replaceable cartridge 9 is used. Otherwise, if the cartridge 9 cannot be replaced, the pen with the present structure may be used only once.

To set a dose, the patient needs to rotate the rotatable wheel in the syringe pen until a graduation indicative of a required dose appears in the window. When the rotatable wheel is rotated in relation to the housing 1 of the syringe pen, the dose-setting mechanism 2 transmits the rotation to the drive 3 and to the adjusting ring 6. Meanwhile, the projections 7 on the adjusting ring 6 are intermeshed with the recesses 8 on the rod 4, resulting in generation of a tactile signal (i.e. vibration) or audible clicks corresponding to the setting of the injection dose.

To adjust the set dose having an excessive volume, the patient needs to rotate the dose-setting mechanism 2 in an opposite direction. When the projections 7 of the adjusting ring 6 are straight, the projections 7 will engage with the recesses 8 on the rod 4 in both directions of rotation.

A signal will be generated both when increasing and decreasing the injection dose. When the projections 7 are curved, a sound signal is provided only for a direction corresponding to the direction of the projections 7.

When the dose-setting mechanism 2 is unscrewed from the housing 1 of the syringe pen when setting the injection dose, the cylindrically shaped drive 3 extends from the housing 1 in the longitudinal direction for a corresponding length. Movement of the dose-setting mechanism 2 and the cylindrically shaped drive 3 in the longitudinal direction in relation to the housing 1 is provided due to the engagement between the dose-setting mechanism 2 and a part of the cylindrically shaped drive 3.

When the required dose is set, the patient presses the push button. The dose-setting mechanism 2 enters the housing 1 of the syringe pen subject to the force applied by the patient when pressing the push button. When the dose-setting mechanism 2 is pushed into the housing 1, the movement is transferred to the cylindrically shaped drive 3 due to the engagement therebetween.

Down movement of the drive 3 as provided when injecting the dose is transferred to the dose delivery screw 5.

Rotation of the dose delivery screw 5 when performing the dose injection results in that the thread of the dose delivery screw 5 mates with the thread of the rod 4, thereby allowing the rod 4 to move in the longitudinal direction for a required length in order to deliver the set injection dose. Therefore, simplified production of the parts of the ratchet mechanism provides an injection syringe pen with a simplified structure. Meanwhile, accuracy of injection dose setting is increased due to the precise intermeshed connection between the projections of the adjusting ring and the recesses, resulting in an improved sound and tactile effect when setting the dose.

This device may be used not only for injecting drugs comprising insulin or its analogs, but also for injecting other drugs comprising hormones, heparins, antihistamines, and their derivatives and analogs.

## Claims

1. An injection syringe pen, comprising:
a housing provided with a push button;
a dose-setting mechanism connected to the push button, wherein the dose-setting mechanism is installed in the housing so as to be longitudinally movable in relation thereto;
a cylindrically shaped drive installed inside the dose-setting mechanism so as to be longitudinally movable in relation to the housing, wherein the cylindrically shaped drive is engaged with the dose-setting mechanism;
a rod installed in the cylindrically shaped drive;
a dose delivery screw installed inside the rod; and
an adjusting ring, wherein the adjusting ring is engaged with the dose-setting mechanism and provided with projections on an inner side, the adjusting ring is further rotatably mounted on the rod, and
wherein recesses are provided on an outer surface of the rod, and the adjusting ring is mounted on the rod such that the projections of the adjusting ring and the recesses of the rod form a ratchet mechanism.

2. The injection syringe pen according to claim 1, wherein the adjusting ring is provided with at least two projections.

3. The injection syringe pen according to claim 1, wherein the projections of the adjusting ring are straight.

4. The injection syringe pen according to claim 1, wherein the projections of the adjusting ring are curved.

5. The injection syringe pen according to claim 1, wherein the projections of the adjusting ring are sharp.

6. The use of the injection syringe pen according to any one of claims 1-5 as a medical product for injecting a pharmaceutical formulation into a human or animal body.

7. The use of the injection syringe pen according to any one of claims 1-5 as a medical product for injecting a drug selected from a group consisting of insulins, insulin analogs, hormones, heparins, antihistamines, and their derivatives and analogs.
